# EUROPÄISCHE PATENTSCHRIFT

(11) **EP 2 683 373 B1**
(45) Veröffentlichungstag und Bekanntmachung des Hinweises auf die Patenterteilung: **25.10.2023**
(21) Anmeldenummer: 12708816.9
(22) Anmeldetag: 07.03.2012
(51) Int. Cl.: A61K 31/198, A61K 33/06, A61K 33/30, A61K 31/4415, A61K 31/51, A61K 31/525, A61K 31/4406, A61K 31/4188, A61K 31/519, A61K 31/409, A61P 25/32, A23L 33/16, A23L 33/175, A23L 33/15, A23L 33/155, A61K 9/00

(54) **ORTHOMOLEKULARES MITTEL GEGEN DIE FOLGEN VON ALKOHOLKONSUM**
ORTHOMOLECULAR AGENT FOR COUNTERING THE CONSEQUENCES OF ALCOHOL CONSUMPTION
AGENT ORTHOMOLÉCULAIRE UTILISÉ CONTRE LES CONSÉQUENCES DE LA CONSOMMATION D'ALCOOL

(30) Priorität: 07.03.2011 DE 102011013224
(43) Veröffentlichungstag der Anmeldung: 15.01.2014
(73) Patentinhaber: Gerdes, Roman, 90482 Nürnberg (DE)
(72) Erfinder: Gerdes, Roman, 90482 Nürnberg (DE)
(74) Vertreter: Wetzel, Fritz
(86) Internationale Anmeldenummer: PCT/EP2012/053900
(87) Internationale Veröffentlichungsnummer: WO 2012/120036

(56) Entgegenhaltungen:
- US-A1- 2007 202 215
- US-A1- 2008 075 710

## Beschreibung

Die vorliegende Erfindung betrifft eine Zusammensetzung sowie eine pharmazeutische Zusammensetzung, insbesondere zur Reduzierung und/oder Vorbeugung von schädlichen Wirkungen von Alkoholkonsum sowie zur Behandlung von Alkoholintoxikationen.

Alkoholische Getränke spielen in der Gesellschaft bekannterweise seit jeher eine mehr oder weniger große und sozial relevante Rolle, die allgemein bekannt ist. Alkoholische Getränke dienen unter anderem der Entspannung, der Erheiterung und der sozialen Enthemmung und fördern insofern häufig soziale Kontakte. Nicht zuletzt zeichnen sich viele alkoholische Getränke durch einen subjektiv als angenehm empfundenen Geschmack aus. Alkoholische Getränke bzw. Alkohol (Ethanol) werden bei vielen gesellschaftlichen Anlässen getrunken, sowohl zu privaten und kulturellen Anlässen als auch zu geschäftlichen Anlässen, beispielsweise bei Geschäftsessen oder Veranstaltungen. Alkohol wird oft spontan und üblicherweise abends getrunken.

Zu bedenken ist allerdings, dass Alkohol nicht nur ein Genussmittel ist, sondern auch ein Suchtmittel darstellt. Und auch wenn Alkohol nicht regelmäßig eingenommen wird, wird er doch, wenn zu sich genommen, häufig in zu großen Dosen eingenommen.

Nach derzeitigem wissenschaftlichem Erkenntnisstand können alkoholische Getränke in geringen Mengen hinsichtlich bestimmter Aspekte gesundheitsförderlich sein (allerdings oftmals wegen anderer nicht-alkoholischer Stoffe, bspw. Resveratrol in Rotwein), in hohen Mengen sind alkoholische Getränke bzw. ist Alkohol allerdings nach derzeitigem wissenschaftlichen Stand in jedem Fall gesundheitsschädlich. Alkohol und dessen erstes Metabolisierungsprodukt Acetaldehyd sind für den Körper als toxisch und körperstressig einzustufen, insbesondere das Acetaldehyd. Die Leber als Entgiftungsorgan wird in hohem Maße belastet und je nach Art und Umfang des Alkoholkonsums entsprechend geschädigt. Ferner steigert Alkoholkonsum das Risiko für viele Tumorerkrankungen, für Herz-Kreislauf-Erkrankungen und für Lebererkrankungen. Alkohol führt zur übermäßigen Ausscheidung und zum übermäßigen Verbrauch von für zahlreiche Stoffwechselprozesse wichtigen Mikronährstoffen. Alkohol führt im Übrigen zu so genanntem oxidativen Stress.

Der toxische Alkohol wird vor allem durch das Enzym Alkoholdehydrogenase (ADH) in das äußerst giftige erste Metabolisierungsprodukt Acetaldehyd umgewandelt. Acetaldehyd wird dann vor allem in Essigsäure verwandelt durch das Enzym Acetaldehyddehydrogenase, kann aber auch von Glutathion und Cystein gebunden werden. Abgesehen vom Alkohol und Acetaldehyd, die beide für sich schon körperstressig sind, führt Alkoholkonsum zu einem körperstressigen Mikronährstoffverlust und einem erhöhten Mikronährstoffbedarf: Auf der einen Seite führt Alkoholkonsum zu einer Zerstörung von Vitaminen und zu einem übermäßigen renalen Ausstoß von Mineralien, Spurenelementen und Vitaminen, auf der anderen Seite entsteht vor allem durch den Alkoholstoffwechsel ein wesentlich erhöhter Mikronährstoffbedarf, den der Körper nicht durch normale Ernährung abdecken kann. Zudem ist in der Regel eine Dehydrierung durch einen für die Alkoholentgiftung überhöhten Wasserverbrauch und Wasserverlust zu bemerken.

Eine allseits bekannte und spürbare Folge bzw. schädliche Wirkung übermäßigen Alkoholkonsums, besonders bei unregelmäßigem Trinken, ist der so genannte Kater (Hangover), den eine Person angesichts der üblicherweise abendlichen Einnahme von Alkohol meist am nächsten Tag hat. Ein Kater, im Grunde eine Vergiftungserscheinung, zeichnet sich durch körperliches und mentales Unwohlsein aus. Eine Person, die einen Kater hat, fühlt sich beispielsweise üblicherweise erschöpft, benommen, kognitiv eingeschränkt, träge, unkonzentriert, reizbar, dysphorisch, niedergeschlagen und ängstlich/unruhig; sie verspürt Körperschmerzen, Kopfschmerzen und Übelkeit, hat oftmals eine verlangsamte Schärfeleistung der Augensicht und weist zudem üblicherweise einen deutlich erweichten Stuhlgang auf.

Ein Kater resultiert vor allem aus den toxischen und körperstressigen Auswirkungen des Alkohols und insbesondere des Acetaldehyds, aber eben auch aus einem alkoholinduzierten Mikronährstoffverlust und erhöhtem Mikronährstoffbedarf und den daraus resultierenden körperstressigen Folgen. Auch die alkoholinduzierte Dehydrierung ist mitverantwortlich für den Kater. Acetaldehyd wird als eine wesentliche Ursache eines Katers gehandelt. Die üblicherweise mit dem Kater verbundenen verminderten kognitiven Fähigkeit und mentalen Verstimmungen sind auch auf Vitamin-, Mineralien- und Spurenelementverlust in Kombination mit einem durch Alkoholkonsum vorübergehend gestörten Neurotransmittergleichgewichts zurückzuführen, sowie auf alkoholinduzierten Schlafmangel.

verminderten Produktivität einer von einem Kater betroffenen Person - und den gesundheitlicher Folgen - sowohl für den Einzelnen und natürlich volkswirtschaftlich insgesamt relevant und werden in verschiedenen Ländern politisch thematisiert.

Gegen den "Kater" werden seit längerem diverse Hausmittel angepriesen, beispielsweise die Verwendung von Aspirin (Acetylsalicylsäure) in Kombination mit Kaffee (Koffein), das Bayerische "Konterbier" (also den Kater mit Alkohol bekämpfen) oder bestimmte Nahrungsmittel. Hilfreich sind diese Hausmittel meist nicht und wenn überhaupt, dann nur in einem sehr geringen Maß.

Besonders in den USA gibt es diverse kommerziell erhältliche Mittel gegen den "Kater", welche jedoch oft nur eine ungezielte Ansammlung verschiedener Mikronährstoffe darstellen und wobei zu bemerken ist, dass diese Mittel grundsätzlich zu niedrig dosiert und/oder in der Wirkstoffkombination nicht ausgewogen sind. Diese Zusammensetzungen unterscheiden sich in der Regel nicht von handelsüblichen selektiven nichttherapeutischen Multivitamin/Multimineral-Präparaten. Hilfreich sind auch diese Mittel meist nicht und wenn überhaupt, dann nur in einem geringen Maß.

Zu erwähnen wäre an dieser Stelle auch Mariendistel (Silymarin), das in erster Linie dafür bekannt ist, toxische Leberschäden bei Medikamenteneinnahme und Alkoholverzehr angeblich zu verringern bzw. rückgängig zu machen. Es zeigt sich jedoch keine besondere Wirkung, um die Folgen eines übermäßigen Alkoholkonsums zu verhindern, zu beseitigen oder zu reduzieren.

Die WO 2010/118405 A2 und US 2010/0316735 A1 offenbaren ein Anti-Hangover-Mittel umfassend einen Pflanzenextrakt, die Mineralstoffe Natrium, Kalium und Magnesium und die Vitamine C, B1, B2, B3, B6 und B12.

US 2008/0075710 A1 offenbart ein pflanzliches Mittel zur Reduzierung der Hangover-Symptome, wobei das Mittel Kalium, Thiamin (Vitamin B1), Magnesium, Silymarin und Acetylcystein enthält. Die Zusammensetzung kann darüber hinaus Vitamin C, Vitamin E, Zink und Folsäure enthalten.

US 2005/0271754 A1 offenbart eine Zusammensetzung zur Verringerung der Hangover-Symptome, wobei die Zusammensetzung Succinsäure, Fumarsäure, eine Unterkomponente des Tripeptids Glutathion, L-Cystein, Saft von jungen Gerstentrieben, Folsäure, Vitamin B1 und B12 sowie eine zuckerbasierte Energiequelle enthält.

US 2004/0248819 A1 offenbart ebenfalls eine Zusammensetzung zur Reduzierung von Hangover-Symptomen, wobei die Zusammensetzung Fructose und ein Fructose enthaltendes Oligosaccharid umfasst. Weitere Bestandteile können Aminosäuren, Vitamine und diverse Mineralstoffe sein.

US 4,496,548 offenbart ebenfalls eine Zusammensetzung zur Reduzierung von Hangover-Symptomen, wobei die Zusammensetzung Ascorbinsäure, Thiamin, Cystein oder Cysteinsäure und ein Flavanoid enthält.

US 2007/202215 A1 offenbart eine Zusammensetzung, welche Vitamin B2, B6, B12, B3, B1, Calcium, Magnesium, Vitamin A, Vitamin C, Folsäure, L-Cystein, L-Methionin, Mariendistel, Selen, Löwenzahn und Molybdän enthält.

Alle im Stand der Technik genannten Zusammensetzungen sind nicht zufrieden stellend und auch in der Dosierung der Komponenten nicht perfekt ausgewogen und unzureichend.

Es besteht also weiterhin ein Bedarf für ein Mittel, das die schädlichen Wirkungen von Alkoholkonsum, insbesondere den Kater/Hangover, verhindert, reduziert oder beseitigt.

Die Aufgabe der vorliegenden Erfindung bestand deshalb in der Bereitstellung einer solchen Zusammensetzung. Die Aufgabe wurde angegangen insbesondere unter Berücksichtigung der Orthomolekularmedizin (d.h. unter anderem auch hinsichtlich eines "orthomolekularen Dosierungsspektrums"), mit Hinblick auf die biochemischen Grundlagen der Stoffwechselprozesse, in diesem Fall insbesondere hinsichtlich der biochemischen Auswirkungen von Alkohol auf den Körper und der genauen Analyse des Alkoholstoffwechsels.

Die Aufgabe wurde gelöst durch eine Zusammensetzung zur Reduzierung und/oder Vorbeugung von schädlichen Wirkungen von Alkoholkonsum umfassend 600-4000 mg einer Cysteinverbindung, ein pharmazeutisch annehmbares Magnesiumsalz, ein pharmazeutisch annehmbares Zinksalz, Vitamin B1, Vitamin B2, Vitamin B3, Vitamin B5, Vitamin B6 und Vitamin B12, wobei die Cysteinverbindung N-Acetylcystein ist.

Ferner kann die Zusammensetzung zusätzlich ein pharmazeutisch annehmbares Calciumsalz und/oder Kupfer und/oder Selen und/oder Mangan und/oder Kalium und/oder Eisen und/oder Jod und/oder Molybdän und/oder Chrom enthalten.

Gemäß einer weiteren Ausführungsform ist es zudem bevorzugt, dass die Zusammensetzung zusätzlich Vitamin C und/oder Vitamin D3 und/oder Vitamin E und/oder Vitamin K1 und/oder Vitamin A und/oder Biotin enthält.

Darüber hinaus kann die Zusammensetzung zusätzlich Methionin und/oder Taurin und/oder Tyrosin und/oder Folsäure und/oder Cholin und/oder Coenzym Q10 und/oder Inositol und/oder Glycin und/oder Glutamin und/oder Glutaminsäure und/oder alpha-Liponsäure enthalten.

Darüber hinaus ist es besonders bevorzugt, dass die Zusammensetzung in einer Dosisform 650-4000 mg, mehr bevorzugt 700-1200 mg, noch mehr bevorzugt 750-1100 mg, am meisten bevorzugt 800-900 mg N-Acetylcystein (NAC) enthält.

Gemäß einer weiteren Ausführungsform der Erfindung kann die Zusammensetzung Piracetam enthalten.

Gemäß einer Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin K1, Vitamin A, Vitamin E, Vitamin D3, Calcium, Kupfer, Selen, Mangan, Kalium, Eisen, Jod, Molybdän, Chrom, Niacin und Coenzym Q10.

Gemäß einer weiteren Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin K1, Vitamin A, Vitamin E, Calcium, Kupfer, Selen, Mangan, Eisen, Molybdän, Niacin, Taurin, Tyrosin und Coffein.

Gemäß noch einer weiteren Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin A, Vitamin E, Calcium, Kupfer, Selen, Mangan, Eisen, Molybdän, Niacin, Methionin, Inositol, Cholin, Glutamin und Carnitin.

Gemäß noch einer weiteren Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin E, Calcium, Kupfer, Selen, Mangan, Kalium, Eisen, Jod, Molybdän, Chrom, Taurin, Cholin, Coen. Q10, Glycin, Glutaminsäure und Alpha-Liponsäure.

Gemäß noch einer weiteren Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin K1, Vitamin A, Vitamin E, Vitamin D3, Calcium, Kupfer, Selen, Mangan, Kalium, Eisen, Jod, Molybdän, Chrom, Niacin, Taurin, Methionin, Tyrosin, Inositol, Cholin, Coen. Q10, Glycin, Glutamin, Glutaminsäure, Alpha-Liponsäure, Coffein und Carnitin.

Gemäß noch einer weiteren Ausführungsform umfasst die Zusammensetzung NAC, Magnesium, Zink, Vitamin C, Vitamin B1, Vitamin B2, Vitamin B3 als Niacinamid, Vitamin B5, Vitamin B6, Vitamin B12, Biotin, Folsäure, Vitamin K1, Vitamin A, Vitamin E, Vitamin D3, Calcium, Kupfer, Selen, Mangan, Kalium, Eisen, Jod, Molybdän, Chrom, Niacin, Taurin, Methionin, Tyrosin, Inositol, Cholin, Coen. Q10, Glycin, Glutamin, Glutaminsäure, Alpha-Liponsäure, Coffein, Carnitin und Piracetam.

Die Dosierungen einzelner Substanzen liegen wegen der auf die Lösung des Problems gezielten Herangehensweisen meist über der *recommended daily allowance* (RDA) (die RDA ist für therapeutische Zwecke nicht relevant), aber bevorzugt unterhalb des jeweiligen NOAEL (no observed adverse effects level), die jeweils unter wissenschaftlichen Konsens festgestellt sind. Die Dosierung ist bevorzugt für einen männlichen Homo Sapiens mit einem Körpergewicht von 75 kg ausgelegt. Männer mit höherem oder niedrigerem Körpergewicht sowie Frauen können entsprechend vom Dosisspektrum angepasst werden, wobei dies nicht notwendig ist.

Im Sinne der Erfindung ist es bevorzugt, dass Vitamin B12 mindestens 100 %, bevorzugt mindestens 200 %, insbesondere mindestens 1500 %, am meisten bevorzugt mehr als 5000 % über RDA liegt (RDA Vit. B12 etwa 2-3 µg bei einem erwachsenen Menschen). Überraschend wurde gefunden, dass eine hohe Dosierung von Vitamin B12 in Verbindung mit einer hohen Dosierung einer Cysteinverbindung, bevorzugt N-Acetylcystein, besonders vorteilhaft ist bei der Reduzierung und/oder Vorbeugung von schädlichen Wirkungen von Alkoholkonsum. Insbesondere sollten alle Nähr- und Vitalstoffe (also Vitamine, Biotin, Folsäure usw.) mindestens 100 %, bevorzugt 150 % über RDA eingesetzt werden, um die positiven Effekte bei der Reduzierung und/oder Vorbeugung von schädlichen Wirkungen von Alkoholkonsum zu steigern.

Der positive Effekt zeigt sich insbesondere auch bei akuten Alkoholintoxikationen durch einen beschleunigten Alkoholabbau. Dadurch eignet sich die erfindungsgemäße Zusammensetzung auch als intravenös verabreichbare Zusammensetzung, beispielsweise als Infusionslösung für die Akutbehandlung von Alkoholintoxikationen.

Die weiteren Komponenten liegen sofern vorhanden in folgenden Mengen in einer Dosisform der Zusammensetzung vor:

| | **Komponente** | **Einheit** | **Mögliche Dosierung** | **Bevorzug te Dosierung** | **Am meisten bevorzugte Dosierung** |
|---|---|---|---|---|---|
| | Magnesium | mg | 100-950 | 300-950 | 300-700 |
| | Zink | mg | 10-150 | 25-150 | 25-125 |
| Vit | C | mg | 500-2000 | 500-1500 | 500-1000 |
| Vit | B1 | mg | 1-200 | 10-200 | 50-100 |
| Vit | B2 | mg | 1 bis 200 | 10-100 | 20-40 |
| Vit | B3 als Niacinamid | mg | 100-1500 | 400-1500 | 500-1300 |
| Vit | B5 | mg | 5-500 | 10-100 | 50-80 |
| Vit | B6 | mg | 10-200 | 25-150 | 50-80 |
| Vit | B12 | µg | 2 bis 2000 | 20-1000 | 500-1000 |
| Vit | Biotin | µg | 50 bis 2000 | 300-1000 | 400-600 |
| Vit | Folsäure | µg | 200-1000 | 400-1000 | 600-800 |
| Vit | K1 | µg | 40-360 | 80-240 | 120-180 |
| Vit | A | µg | 400-2000 | 400-1600 | 400-1000 |
| Vit | E | mg | 10-800 | 10-800 | 100-300 |
| Vit | D3 | µg | 1 bis 50 | 1 bis 50 | 1-5 |
| | Calcium | mg | 50-1500 | 50-1500 | 200-600 |
| | Kupfer | mg | 1 bis 10 | 1 bis 10 | 2 bis 5 |
| | Selen | µg | 10-800 | 25-400 | 50-100 |
| | Mangan | mg | 1 bis 10 | 1 bis 10 | 2 bis 3 |
| | Kalium | mg | 20-4000 | 20-4000 | 20-50 |
| | Eisen | mg | 2 bis 45 | 2 bis 45 | 5-40 |
| | Jod | µg | 50-1000 | 100-450 | 100-250 |
| | Molybdän | µg | 20-200 | 25-100 | 40-60 |
| | Chrom | µg | 10-1000 | 20-500 | 20-60 |
| Vit | Niacin | mg | 5-500 | 5-100 | 10-40 |
| | Taurin | mg | 100-4000 | 100-3000 | 500-1000 |
| | Methionin | mg | 100-4000 | 250-1000 | 400-600 |
| | Tyrosin | mg | 50-5000 | 250-1000 | 400-600 |
| | Inositol | mg | 10 bis 20000 | 200-1500 | 200-300 |
| | Cholin | mg | 50-10000 | 200-1200 | 200-300 |
| | Coen. Q10 | mg | 1 - 400 | 1 -30 | 2-10 |
| | Glycin | mg | 1-1000 | 100-800 | 400-500 |
| | Glutamin | mg | 10-40000 | 500-2000 | 800-1200 |
| | Glutaminsäure | mg | 10 - 2400 | 200-1200 | 400-600 |
| | Alpha-Liponsäure | mg | 20-1200 | 50-300 | 40-60 |

Alle Mengenangaben beziehen sich auf die Menge des Wirkstoffs als solchen. D.h. bei einem Salz, beispielsweise einem Magnesium-, Zink- oder Calciumsalz, auf die Gewichtsmenge des Magnesiumions, Zinkions oder Calciumions in der Gesamtverbindung. Bei organischen Verbindungen, die beispielsweise salzartig als Hydrochlorid oder Sulfat vorliegen, nur auf die Gewichtsmenge der Hauptverbindung und nicht zusätzlich auch auf das Gegenion. Die bevorzugten Dosierungen beziehen sich insbesondere auf eine einzelne Dosisform. Darüber hinaus versteht es sich von selbst, dass auch eine vielfache Menge einer Einzeldosis für eine Zusammensetzung als unter Schutz gestellt gilt. Hierbei gilt, dass die Dosis der einzelnen Komponenten entsprechend in Gew.-%, bezogen auf die Zusammensetzung umgerechnet wird.

Insbesondere ist es vorteilhaft, die erfindungsgemäße Zusammensetzung, wenn sie nicht als Fertigflüssigkeit angeboten wird, immer mit einer ausreichenden Menge an Lösungsmittel, insbesondere Wasser, zu verabreichen, um den Dehydrierungseffekt der durch den übermäßigen Alkoholkonsum verursacht wurde, zu kompensieren. Beispielsweise sollte ein Granulat idealerweise in 300-500 ml Wasser aufgelöst werden bzw. entsprechende Darreichungsformen wie bspw. Pulver, Granulate, Kapseln usw. idealerweise mit 300-500 ml und mehr Wasser eingenommen werden.

Gemäß einer weiteren Ausführungsform der erfindungsgemäßen Zusammensetzung ist es zudem bevorzugt, dass die Zusammensetzung bereits zusätzlich Wasser enthält. Die Zusammensetzung ist dadurch eine flüssige Formulierung und kann als Trinklösung oder auch als Infusionslösung zur intravenösen Verabreichung eingesetzt werden. Die Menge an Wasser erfüllt hier insbesondere den Zweck, den durch einen Alkoholmissbrauch hervorgerufenen Dehydrierungseffekt zu kompensieren. Bei einer Trinklösung liegen die vorteilhaften Mengen an, die im Sinne dieser Erfindung somit bevorzugt sind, beispielsweise im Bereich von 20 ml bis 1000 ml, mehr bevorzugt 100 bis 1000 ml, noch mehr bevorzugt 200 bis 1000 ml, welche der erfindungsgemäßen Zusammensetzung zugesetzt werden. Besonders vorteilhaft ist auch eine Menge von etwa 500 ml Wasser.

Überraschend wurde gefunden, dass durch eine erfindungsgemäße Zusammensetzung, wie oben beschrieben, die schädlichen Wirkungen, welche durch den Konsum von Alkohol hervorgerufen werden, reduziert werden können bzw. sogar nahezu verhindert oder beseitigt werden können. Dies konnte durch die Auswahl der einzelnen Komponenten in ihrer Zusammenstellung in der erfindungsgemäßen Zusammensetzung erreicht werden. Insbesondere fördernd für die Lösung der erfindungsgemäßen Aufgabe ist es, wenn die erfindungsgemäß eingesetzten Komponenten in der Zusammensetzung in der beschriebenen Dosis verabreicht werden.

Im Sinne dieser Erfindung wird unter dem Begriff "schädliche Wirkungen" eine ganze Reihe von negativen Folgen von übermäßigem Alkoholkonsum verstanden, die man grob in unmittelbare Folgen und Folgekrankheiten unterteilen kann und die im Folgenden ausführlich, aber ohne Anspruch auf Vollständigkeit, ausgezählt werden: Unmittelbare Folgen sind beispielsweise der Kater, vor allem wegen der Alkohol-"vergiftung" und Acetaldehyd-"vergiftung". Insofern kommt es unter anderem zu einem Anstieg des Alkoholblutspiegels, einer Überlastung der Leber, Mikronährstoffverlust, -zerstörung und -verbrauch, (übermäßige) Dehydrierung, depressiver Verstimmung, Unruhe und Ängstlichkeit, körperlichem Unwohlgefühl, Übelkeit, Appetitverlust, kognitiver Einschränkung, Konzentrationsstörungen, Kopfschmerzen und anderes. Die Folgekrankheiten, insbesondere bei regelmäßigem Alkoholkonsum, sind beispielsweise Mikronährstoffverlust, - zerstörung und -verbrauch (übermäßig) und alle daraus folgenden Körpermissstände, auch Stoffwechselstörungen. Körperstressige Wirkungen von Alkohol und Acetaldehyd sind unter anderem eine Schädigung der Leber und anderer Organe, ein erhöhtes Krebsrisiko, eine Fettleber, eine Leberzirrhose und eine grundsätzliche "Überlastung" der Leber, aber auch physische und psychische Missstände in Folge von andauerndem Mikronährstoffmangel. Ferner besteht das Risiko eines Gewöhnungseffekts, einer Sucht, von neurologischen Schädigungen und vielem mehr.

Die erfindungsgemäße Zusammensetzung kann die oben genannten schädlichen Wirkungen des Alkoholkonsums verringern oder beseitigen durch einen besseren Abbau der Giftstoffe Alkohol und Acetaldehyd. Es kommt zu einem "Nachfüllen" der durch Alkohol verloren gegangenen Mikronährstoffe (Vorbeugung / Linderung eines alkoholinduzierten Mikronährstoffmangels), einer Beschleunigung des Abbaus von Alkohol bzw. Verhinderung entsprechender Intoxikation bei gleicher Menge Alkohol, einem Schutz der Leber, einer Unterstützung der Leber, einem schnelleren Nüchtern werden, einer Reduzierung eines erhöhten Homocysteinspiegels durch Alkohol und Schutz vor oxidativem Stress, Linderung und Vorbeugung depressiver Verstimmung, Antriebs- und Konzentrationsstörungen am Folgetag (Neurotransmitter) sowie einer Beseitigung der Katersymptome.

Die einzelnen Komponenten der Zusammensetzung werden, sofern nichts anderes genannt ist, in handelsüblichen und für den pharmazeutischen Bereich oder Lebensmittelbereich üblichen und zugelassenen Formen verwendet.

Pharmazeutisch annehmbare Salze für alle im Rahmen dieser Erfindung genannten Verbindungen sind, falls nichts anderes bei einer entsprechenden Komponente genannt ist, bevorzugt ausgewählt aus Chlorid, Bromid, Iodid, Sulfat, Phosphat, Tartrat, Acetat, Mucat, Citrat, Aspartat und Orotat. Die Hydrate sind bevorzugt ausgewählt aus Mono-, Di-, Tri-, Tetra-, Penta- und Heptahydrat.

Gegebenenfalls sind der erfindungsgemäßen Zusammensetzung noch pharmazeutisch annehmbare Träger sowie Hilfs- und Zusatzstoffe beigemischt.

Pharmazeutisch annehmbare Träger sowie Hilfs- und Zusatzstoffe sind beispielsweise ein Feststoff oder eine Flüssigkeit. Beispiele für feste Träger sind unter anderem Lactose, Porzellanerde, Saccharose, Talk, Gelatine, Agar, Pektin, Akaziengummi, Magnesiumstearat und Stearinsäure. Beispiele für flüssige Träger sind Zuckersirup, Erdnussöl, Olivenöl und Wasser. Beispiele für Hilfs- und Zusatzstoffe sind Brausemittel, bevorzugt Natriumhydrogencarbonat und Citronensäure, sowie Verdünnungsmittel, Puffer, Granuliermittel, Gleitmittel, Sprengmittel, Bindemittel, oberflächenaktive Mittel, Verdickungsmittel sowie Farbstoffe und Pigmente, Konservierungsstoffe (einschließlich Antioxidantien), Geschmacksstoffe und Aromastoffe.

Gemäß einer weiteren Lösung der erfindungsgemäßen Aufgabe kann die Zusammensetzung auch für einen Langzeitgebrauch konzipiert sein, etwa für Personen, die regelmäßig oder chronisch Alkohol konsumieren und insofern entsprechend therapeutisch dosierte Mikronährstoffe benötigen, um die schädlichen Wirkungen von Alkoholkonsum zu reduzieren (wobei in dieser Ausführung also nicht in erster Linie die Verhinderung, Linderung und/oder Beseitigung des Katers bei nicht-chronischen Alkoholkonsumenten im Vordergrund steht). Dazu ist es bevorzugt, die Dosisbereiche der einzelnen Komponenten zu reduzieren, insbesondere mit Hinblick auf die durch wissenschaftlichen Konsens jeweils ermittelten UL ((daily) tolerable Upper intake Level / safe Upper Limit).

Für die Zusammensetzung für eine Langzeitanwendung ergeben sich folgende Dosierungen der einzelnen Komponenten:

| | **Komponente** | **Einheit** | **Mögliche Dosierung** | **Bevorzugte Dosierung** | **Am meisten bevorzugte Dosierung** |
|---|---|---|---|---|---|
| | Cysteinver bindung | mg | 10-4000 | 10-600 | 100-300 |
| | Magnesium | mg | 10-400 | 40-400 | 150-400 |
| | Zink | mg | 1-50 | 10-45 | 20-45 |
| Vit | C | mg | 50-2000 | 200-150 | 200-1000 |
| Vit | B1 | mg | 0,1-200 | 1-100 | 5-75 |
| Vit | B2 | mg | 0,1 bis 200 | 1-100 | 2-50 |
| Vit | B3 als Niacinamid | mg | 10-900 | 40-800 | 50-600 |
| Vit | B5 | mg | 0,5-1000 | 1-100 | 5-50 |
| Vit | B6 | mg | 1-150 | 2,5-100 | 5-50 |
| Vit | B12 | µg | 0,2 bis 2000 | 2-1000 | 50-500 |
| Vit | Biotin | µg | 5 bis 2000 | 30-1000 | 40-300 |
| Vit | Folsäure | µg | 20-1000 | 40-800 | 60-400 |
| Vit | K1 | µg | 4-360 | 10-200 | 10-100 |
| Vit | A | µg | 40-2000 | 40-800 | 40-400 |
| Vit | E | mg | 1-800 | 10-600 | 10-300 |
| Vit | D3 | µg | 0,1 bis 50 | 0,1 bis 25 | 5-25 |
| | Calcium | mg | 5-1500 | 5-1000 | 30-800 |
| | Kupfer | mg | 0,1 bis 10 | 1 bis 5 | 1 bis 2 |
| | Selen | µg | 1-400 | 2,5-200 | 5-50 |
| | Mangan | mg | 0,1 bis 11 | 0,1 bis 10 | 0,2 bis 2,0 |
| | Kalium | mg | 2-4000 | 2-2000 | 10-500 |
| | Eisen | mg | 0,2 bis 45 | 10 bis 35 | 10-25 |
| | Jod | µg | 5-1000 | 10-450 | 10-250 |
| | Molybdän | µg | 2-200 | 2,5-100 | 10-100 |
| | Chrom | µg | 1-1000 | 2-500 | 10-100 |
| | Niacin | mg | 0,5-200 | 0,5-100 | 1-50 |
| | Taurin | mg | 10-4000 | 10-3000 | 50-1000 |
| | Methionin | mg | 10-4000 | 25-1000 | 40-600 |
| | Tyrosin | mg | 5-5000 | 25-1000 | 40-600 |
| | Inositol | mg | 1 bis 2000 | 20-1500 | 20-300 |
| | Cholin | mg | 5-1000 | 20-1200 | 20-300 |
| | Coen. Q10 | mg | 0,1 - 40 | 0,1 -30 | 0,2-10 |
| | Glycin | mg | 0,1-1000 | 10-800 | 40-500 |
| | Glutamin | mg | 1-4000 | 50-2000 | 80-1200 |
| | Glutaminsä ure | mg | 1 - 2400 | 20-1200 | 40-600 |
| | Alpha-Liponsäure | mg | 2-400 | 10-200 | 20-100 |

Die erfindungsgemäß eingesetzten Komponenten weisen bereits als Einzelsubstanzen bestimmte physiologische Wirkungen auf, jedoch erst im Zusammenspiel, also in der Kombination der Einzelkomponenten, kann sich die Gesamtwirkung vorteilhaft entfalten.

Beispielsweise ist die erfindungsgemäß eingesetzte Cysteinverbindung N-Acetylcystein (NAC) ein wichtiger Precursor des Peptids Glutathion (bestehend aus Cystein, Glycin und Glutaminsäure), welches ein starkes Antioxidans sowie ein wesentliches Entgiftungsmittel in der Leber darstellt. Glutathion und Cystein bzw. das bevorzugt eingesetzte N-Acetylcystein weisen aufgrund ihrer SH-Gruppe mit Donorwirkung ausgeprägte antioxidative und detoxifizierende Eigenschaften auf.

Glutathion unterstützt die Leber wesentlich bei der Entgiftung bzw. dem Abbau von Alkohol bzw. Acetaldehyd und schützt die Leber vor den toxischen Wirkungen des Acetaldehyds. Unter anderem bindet das schwefelhaltige Glutathion Acetaldehyd. Übermäßiger Alkoholkonsum führt zu einer Reduzierung von Glutathion, insbesondere in der Leber. Auch Cystein bzw. Acetylcystein wirken bei der Entgiftung von Alkohol bzw. Acetaldehyd in der Leber durch Bindung und schützen die Leber somit vor den toxischen Wirkungen des Acetaldehyds.

Problematisch ist jedoch die geringe Resorption und somit geringe Bioverfügbarkeit von Glutathion. In den letzten Jahren hat sich ein wissenschaftlicher Konsens gebildet, dass der Körper Glutathion nur in geringem Maße aufnehmen kann. Insofern ist man darauf angewiesen, dass der Körper selbst Glutathion aus den drei genannten Aminosäuren Glycin, Glutaminsäure und Cystein synthetisiert. Um die Produktion von Glutathion zu stimulieren ist deshalb eine zusätzliche Aufnahme von L-Cystein, beispielsweise in der Form von Acetylcystein hilfreich, wobei eine zusätzliche Gabe von Glycin und Glutaminsäure gewöhnlich nicht erforderlich ist. Die direkte Aufnahme von L-Cystein ist jedoch dahingehend problematisch, da L-Cystein, welches gewöhnlich als Hydrochlorid vorliegt, instabil ist und im Körper häufig in L-Cystin durch Oxidation umgewandelt wird. Für die Bereitstellung von Cystein und der Glutathionproduktion ist somit eine gezielte Zufuhr von L-Cystein insofern offensichtlich wenig effizient und wirksam. Erfindungsgemäß wird deshalb bevorzugt acetyliertes Cystein (N-Acetylcystein) eingesetzt, welches vom Körper gut aufgenommen und verarbeitet wird. N-Acetylcystein wirkt somit effizienter und wirksamer als L-Cystein. Es ist zudem stabiler als L-Cystein und wird in der Leber fast vollständig zu Cystein umgewandelt und insbesondere dort auch zu Glutathion umgesetzt. Somit unterstützt N-Acetylcystein wesentlich den Körper bei der Alkoholentgiftung und ist nicht nur als Reservoir für verlorengegangenes Glutathion anzusehen. Die Verwendung von N-Acetylcystein in der erfindungsgemäßen Zusammensetzung ist somit ein wesentlicher Bestandteil der Erfindung.

Ein weiterer wichtiger Bestandteil der erfindungsgemäßen Zusammensetzung ist Magnesium. Magnesium ist an über 300 Stoffwechselprozessen im Körper beteiligt und reguliert die Reizübertragung in Muskeln und Nerven. Ein Magnesiummangel kann beispielsweise ein Grund für Kopfschmerzen sein. Eine erhöhe Alkoholeinnahme führt unmittelbar zu einer erhöhten renalen Ausscheidung von Magnesium und stört die Resorption und führt somit zu einem temporären Mangel. Magnesiummangel ist auch eines der wesentlichen Ursachen für einen Kater. Ein Nachschub von Magnesium ist deshalb sinnvoll und wirkungsreich zur Bekämpfung und/oder Verhinderung eines Katers. Magnesium ist insbesondere auch deshalb vorteilhaft, da es ADP stabilisiert.

Viele Magnesiumeinzelpräparate enthalten jedoch anorganische Magnesiumverbindungen, beispielsweise Oxide und Carbonate, deren Bioverfügbarkeit gering ist. Entscheidend für die erfindungsgemäßen Vorteile der vorliegenden Erfindung ist jedoch eine ausreichende Bioverfügbarkeit der Magnesiumverbindung. Erfindungsgemäß wird deshalb bevorzugt eine organische Magnesiumverbindung, vorzugsweise Magnesiumcitrat (ca. 620 mg Trimagnesiumdicitrat entspricht etwa 100 mg Magnesium), Magnesiumorotat oder Magnesiumaspartat eingesetzt.

Unter Bioverfügbarkeit wird im Sinne dieser Erfindung der Anteil in Prozent bzw. Gewichtsprozent eines Wirkstoffes, der bei oraler Verabreichung der erfindungsgemäßen Zusammensetzung unverändert im Blut erscheint, zu verstehen. (Die Bioverfügbarkeit eines intravenös injizierten Arzneimittels ist definitionsgemäß 100%. Zur weitergehenden Definition wird auf Rainer K. Liedtke, Wörterbuch der Klinischen Pharmakologie, Gustav Fischer Verlag, Stuttgart, New York, 1980 verwiesen. Die Bioverfügbarkeit ist auch im WHO Annex 9, 1996 definiert.)

Das erfindungsgemäß eingesetzte Vitamin C (Ascorbinsäure, Ascorbat) ist als starkes Antioxidans bekannt und stabilisiert zudem Cystein und Glutathion. Bei übermäßigem Alkoholkonsum ist deshalb eine ausreichende Menge an Vitamin C erforderlich. Erfindungsgemäß ist eine Dosierung von ca. 500 bis 1000 mg vorteilhaft.

In der Regel wird bei einer Magnesiumzufuhr auch eine zusätzliche Zufuhr von Calcium empfohlen, welche im Bereich von etwa 1:2 anzusiedeln ist. Der Grund hierfür liegt darin, dass eine Magnesiumzufuhr die Calciumaufnahme stören kann. Calcium ist unter anderem wichtig für seine Funktion in der Reizübertragung im Nervensystem. Eine Calciumzufuhr wirkt zudem einer Stuhlerweichung durch Magnesium entgegen. Ferner wirkt Calcium dem sauren Vitamin C, wenn es als Ascorbinsäure vorliegt, entgegen. Es ist allerdings zu bedenken, dass die Magnesiumzufuhr wegen des erhöhten Verlustes nicht mit der "üblichen" doppelten Menge Calcium ausgeglichen werden muss. Das Calcium wird bevorzugt als Calciumphosphat, Calcium-D-gluconat, Calciumlactat, Calciumcarbonat oder Calciumascorbat eingesetzt.

Zink ist ein wesentliches Element des Enzyms Alkoholdehydrogenase (ADH), wobei pro Molekül ADH etwa 4 Zinkatome benötigt werden. Zink ist insbesondere bei der Entgiftung des Körpers im Allgemeinen vorteilhaft. Erhöhter Alkoholkonsum führt einerseits zu einer erhöhten Zinkausscheidung (renal), andererseits aber auch zu einem erhöhten Bedarf aufgrund der erhöhten Menge an benötigtem ADH. Zink ist an vielen Stoffwechselprozessen beteiligt, auch an der Neurotransmittersynthese (beispielsweise Dopamin). Alkohol führt zu einer temporär überhöhten Dopaminausschüttung und einem darauffolgenden Dopaminmangel, welcher in Depressionen enden kann. Vorteilhaft im Sinne der Erfindung ist eine Dosierung von 10-150 mg, bevorzugt 25-150 mg, am meisten bevorzugt 25-125 mg Zink, vorzugsweise in Form von Zinksulfat, da die daraus resultierende Schwefelgruppe auch für die Bindung von Acetaldehyd sinnvoll sein kann. Höhere Mengen an Zink sind nicht vorteilhaft bzw. führen zu unerwünschten Wirkungen wie bspw. Übelkeit. Insofern ist die finale Dosis bedachtsam abzustimmen, da Zink zwar eben sehr wichtig ist, aber in zu hoher Menge Nachteile mit sich bringt. Zink kann sowohl als anorganisches als auch organisches Salz eingesetzt werden. Bevorzugt ist Zinksulfat in all seinen Verbindungen. Es ist gut verträglich und leicht verfügbar. Ein weiterer Vorteil von Zinksulfat wird, wie oben erwähnt, dadurch bedingt, dass die Verbindung schwefelhaltig ist und insofern eventuell hilfreich beim Acetaldehyd scavenging fungiert.

Das in der erfindungsgemäßen Zusammensetzung eingesetzte Kupfer wird zur Unterstützung des Zinkanteils beigefügt.

Weiterhin ist Selen ein wichtiges Element für die Entgiftung im Körper. Selen ist ein regulatorischer und katalytischer Cofaktor Selenocystein-haltiger Proteine (Enzyme), beispielsweise in der Glutathion-Peroxidase und Thioredoxin-Reduktase, welche u.a. für die Reduktion von Disulfiden zu SH-Gruppen verantwortlich sind. Da Alkohol zudem die Selenaufnahme behindert, ist eine Dosierung von etwa 50-100 mg Selen erfindungsgemäß am meisten bevorzugt.

Alkohol führt gewöhnlich zur Zerstörung bzw. einer Abnahme aller B-Vitamine, sowohl bei erhöhtem unregelmäßigem Alkoholkonsum als auch bei chronischem Alkoholmissbrauch. In der Literatur wird als Ursache von Vitamin-B-Mangel aller Typen unter anderem ein übermäßiger Alkoholkonsum angeführt. Zudem werden B-Vitamine im Körper schlecht gespeichert.

Insofern ist gerade bei Alkoholkonsum ein ausreichender Nachschub von Vitamin B aller Typen sehr wichtig für die körperliche und geistige Befindlichkeit.

B-Vitamine sind für die geistige Leistungsfähigkeit und für viele Körperfunktionen und Stoffwechselprozesse unerlässlich, auch für den Alkoholstoffwechselprozess. Sie sind zudem meist Antioxidantien und für die Aufnahme anderer Mikronährstoffe wichtig und an Stoffwechselprozessen in Verbindung mit anderen Mikronährstoffen beteiligt (beispielsweise Vitamin B1 und Vitamin B6 unterstützen die Magnesiumaufnahme, B6 in Verbindung mit L-Tyrosin die Neurotransmittersynthese, B1 ist für den Neurotransmitterstoffwechsel relevant). Ein typisches Symptom chronischen Alkoholkonsums ist beispielsweise ein Mangel an Vitamin B1. Alkohol erhöht auch den Homocysteinspiegel, der durch Vitamin B6, Folsäure und B12 reduziert werden kann. Grundsätzlich sind die B-Vitamine für die geistige und auch körperliche Befindlichkeit wichtig. Ein, wenn auch nur temporär durch Alkohol bedingter Mangel, führt unter anderem zu Müdigkeit, Konzentrationsstörungen, Antriebsstörungen und depressiver Verstimmung.

Als Vitamin B12 wird bevorzugt Cyanocobalamin eingesetzt, jedoch können auch als Alternative Methylcobalamin, Hydroxycobalamin und Adenosylcobalamin und die dem Fachmann bekannten Verbindungen eingesetzt werden.

Vitamin B1 (bevorzugt als Thiamin) ist im Neurotransmitterstoffwechsel sowie in der Bereitstellung von Glutathion und als Antioxidans beteiligt. Acetaldehyd stört den Thiaminstoffwechsel, weswegen Vitamin B1 vorteilhaft in der erfindungsgemäßen Zusammensetzung vorhanden ist.

Vitamin B2 (bevorzugt als Riboflavin) dient u.a. zur Reduktion von oxidiertem (GSSG) zu reduziertem Glutathion (GSH), d.h. der Regenerierung von Glutathion.

Vitamin B3, bevorzugt als Niacinamid (Nicotinsäureamid), aber auch als Niacin (Nicotinsäure) einsetzbar, dient u.a. zur Reduktion von oxidiertem (GSSG) zu reduziertem Glutathion (GSH), d.h. der Regenerierung von Glutathion, und führt zu einer Erhöhung des NAD-Spiegels in der Leber. NAD ist notwendig für den Alkohol- und Acetaldehyd-Abbau, da es bei ADH und ALDH als Coenzym fungiert, bei Alkoholkonsum besteht insofern ein erhöhter Bedarf. Sowohl Niacinamid als auch Niacin erhöhen den NAD-Spiegel. Im Vergleich zu Niacin ist Niacinamid in relativ höherer Dosis allerdings besser verträglich (eine hohe Dosierung von Niacin führt unter anderem zu "flushing"). Allerdings wird vermutet, dass der Körper bei Bedarf die Aminosäure Tryptophan, einem wichtigen Neurotransmitter-Precursor (wichtig für das geistige Wohlbefinden), in Niacin umwandelt (60mg = 1mg). Eine zusätzliche Niacinzufuhr könnte insofern zudem eine Tryptophanumwandlung einschränken. Die Niacinzufuhr erfolgt bevorzugt in vergleichsweise niedriger Dosierung. Darüber hinaus hat hochdosiertes Niacinamid eine beruhigende Wirkung, was sich angesichts der anregenden Wirkung von Alkohol als vorteilhalt erweisen kann.

Vitamin B5 (bevorzugt als Pantothensäure) unterstützt u.a. ALDH, da aus Cystein und Vitamin B5 das Coenzym A synthetisiert wird, welches als Cofaktor für ALDH dient.

Vitamin B6 (bevorzugt als Pyridoxin, gegebenenfalls als Hydrochlorid (HCl)) ist unter anderem für die Neurotransmittersynthese eine wesentliche Komponente.

Vitamin B9 (bevorzugt als Folsäure) ist u.a. für den Homocysteinstoffwechsel von Bedeutung. Alkohol-induzierter Folsäuremangel kann zudem das Brustkrebsrisiko erhöhen.

Da Alkohol durch die Stimulierung des Cyt-P450 Systems zu einer Beschleunigung des Vitamin-A Abbaus führt, wird der Zusammensetzung Vitamin A beigefügt. Die Dosierung sollte allerdings niedrig bleiben, weil eine zu hohe Dosierung zusammen mit Alkohol negative Folgen haben kann. Vitamin A wird bevorzugt als Vitamin-A-Acetat eingesetzt.

Vitamin E wird bevorzugt als Vitamin E-Acetat (α-Tocopherol) eingesetzt. Das erfindungsgemäß eingesetzte Vitamin E (α-Tocopherol) ist ein zentrales, wichtiges und starkes Antioxidans und insofern bei Alkoholkonsum verbunden mit oxidativem Stress von zentraler Bedeutung. Es verhindert zudem die Destruktion von Vitamin A und β-Carotin durch Alkohol.

Biotin (Vitamin H) kann ebenfalls als Komponente in der erfindungsgemäßen Zusammensetzung vorliegen. Alkohol stört unter anderem die Resorption von Biotin, ein Mangel kann zu depressiver und ängstlicher Verstimmung und auch zu Übelkeit und Durchfall führen.

Alkohol behindert das Recycling von Vitamin K, einem starken Antioxidans. Bevorzugt wird deshalb eine nicht zu hohe Dosierung beigefügt. Eine nicht zu hohe oder relativ niedrige Dosierung oder ähnliches ist in dieser Schrift zu verstehen als relativ zu "orthomolekularen Dosierungen", die üblicherweise weit über dem RDA sind.

Da ein Eisenmangel zu den häufigsten Mikronährstoffmängeln gehört (Müdigkeit ist ein Symptom) und bspw. die Aufnahme von Zink und Calcium den Eisenhaushalt stören kann, wird der Zusammensetzung vorzugsweise Eisen in nicht zu hoher Dosierung beigefügt.

Alkoholmissbrauch zieht einen Mangel bzw. erhöhten Bedarf an Jod nach sich. Insofern wird der Zusammensetzung bevorzugt Jod in nicht zu hoher Dosierung beigefügt. Jod weist zudem antioxidative Eigenschaften aus.

Calcium, Eisen, Zink und Mangan können die Chrom-Resorption stören. Der Zusammensetzung wird deshalb bevorzugt Chrom beigefügt.

Molybdän ist vermutlich am Alkoholstoffwechsel beteiligt. Der Zusammensetzung wird deshalb bevorzugt Molybdän in relativ niedriger Dosierung beigefügt.

Die Zusammensetzung kann ferner Piracetam enthalten. Piracetam ist ein cyclisches Derivat (γ-Lactam) der γ-Aminobuttersäure (GABA) und ein Wirkstoff aus der Gruppe der Antidementiva bzw. Nootropika. Es regt den zellulären Zuckerstoffwechsel und die Sauerstoffverwertung im Gehirn an und wird unter anderem zur symptomatischen Behandlung hirnorganisch bedingter Leistungsstörungen eingesetzt. Erfindungsgemäß bevorzugt ist eine Menge von 100 bis 3600 mg, mehr bevorzugt 400 bis 2400 mg, am meisten bevorzugt 800 bis 1600 mg.

Gemäß einer weiteren Ausführungsform kann die Zusammensetzung auch Acetyl-Glutathion und/oder L-Theanin enthalten.

Als Darreichungsform sollte die erfindungsgemäße Zusammensetzung vorteilhafterweise mit Wasser verabreicht werden, wobei die bei der Zusammensetzung angegebenen Mengen bevorzugt sind. Wenngleich es offensichtlich erscheint, ist Wasser ein wichtiger Bestandteil bei der Reduzierung der schädlichen Wirkungen von Alkoholmissbrauch, da Alkohol aufgrund einer forcierten Diurese zu einer Dehydrierung des Körpers beitragen kann. Vorteilhafterweise sind die in der erfindungsgemäßen Zusammensetzung eingesetzten Komponenten allesamt wasserlöslich, weshalb die erfindungsgemäße Zusammensetzung bevorzugt als wässrige Lösung verabreicht werden sollte.

Die Darreichungsform der erfindungsgemäßen Zusammensetzung kann beispielsweise als Granulat, als Direktgranulat, als Brausetablette, als Pulver, Kapsel, als Ölkapsel (Fest-Flüssig-Kapsel) oder Gelcap, Retardformulierung, Tablette, als Fertigflüssigkeit, als Infusionslösung, als Inhalationsform oder in irgendeiner Kombination der genannten Darreichungsformen verabreicht werden. Dabei können die dem Fachmann für diese Zwecke geeigneten pharmazeutisch annehmbaren Träger sowie Hilfs- und Zusatzstoffe beigemischt sein. Ebenso kann die Zusammensetzung in Form eines Retardpräparats verabreicht werden. Dies ist insbesondere bei einer Langzeitanwendung vorteilhaft.

Die Herstellung der erfindungsgemäßen Zusammensetzung in Form eines Granulats, eines Direktgranulats, einer Brausetablette, eines Pulvers, einer Kapsel, einer Ölkapsel (Fest-Flüssig-Kapsel) oder Gelcap, einer Retardformulierung, einer Tablette, einer Fertigflüssigkeit, einer Infusionslösung, einer Inhalationsform oder in irgendeiner Kombination der genannten Darreichungsformen, jeweils mit verzögerter oder ohne verzögerte Freisetzung, erfolgt nach im Stand der Technik bekannten Verfahren, beispielsweise durch Mischen der Komponenten, gegebenenfalls Zugabe der Träger sowie Hilfs- und Zusatzstoffe, und weiteres Mischen, gegebenenfalls Lösen oder Dispergieren der Komponenten bzw. Verpressen der Komponenten zu einer Tablette.

Vorteilhaft an der erfindungsgemäßen Zusammensetzung ist, dass sie während, vor und unmittelbar nach einem Alkoholkonsum eingenommen werden kann und so bereits die schädlichen Wirkungen des Alkoholkonsums, insbesondere ein Kater, vermieden werden können. Üblicherweise trinken Menschen abends, insofern ist es wichtig, dass die Einnahme vor dem Schlafen mit einer ausreichenden Menge Wasser stattfindet. Die Einnahmemöglichkeit nach dem Alkoholkonsum ist insofern wichtig, als Alkohol oft spontan übermäßig getrunken wird. Nimmt man das Mittel erst am Morgen, kann man durch Einnahme des Mittels den Kater bekämpfen, der Kater verschwindet sehr viel schneller. Eine zusätzliche Einnahme von Taurin in Verbindung mit Koffein kann die Folgen des Katers zudem bekämpfen. Ebenso kann Carnitin in Verbindung mit Koffein die Folgen des Katers bekämpfen. Eine Einnahme von Taurin/Koffein oder Carnitin/Koffein sollte vorteilhafterweise am Tag nach dem Alkoholkonsum erfolgen.

Ein weiterer Gegenstand der vorliegenden Erfindung ist des Weiteren eine Verwendung der erfindungsgemäßen Zusammensetzung als Nahrungsergänzungsmittel (derzeit gemäß EU Richtlinie 2002/46/EG), als diätetisches Lebensmittel (gemäß der Verordnung über diätetische Lebensmittel der BRD) oder als Medikament.

Ein weiterer Gegenstand ist ferner die Verwendung der erfindungsgemäßen Zusammensetzung zur Reduzierung und/oder Vorbeugung von schädlichen Wirkungen von Alkoholmissbrauch.

Ein weiterer Gegenstand der vorliegenden Erfindung ist eine pharmazeutische Zusammensetzung zur Reduzierung und/oder Vorbeugung von schädlichen Wirkungen von Alkoholmissbrauch, wobei die pharmazeutische Zusammensetzung eine Zusammensetzung wie oben beschrieben enthält. Vorzugsweise ist die pharmazeutische Zusammensetzung eine oral verabreichbare Zusammensetzung.

Ein weiterer Gegenstand der vorliegenden Erfindung ist ein Verfahren zur Reduzierung und/oder Vorbeugung von schädlichen Wirkungen von Alkoholmissbrauch, wobei das Verfahren die Verabreichung einer wirksamen Menge einer Zusammensetzung oder pharmazeutischen Zusammensetzung, wie oben beschrieben, an eine bedürftige Person (Mensch) umfasst.

"Wirksame Menge" ist dabei eine Menge der Zusammensetzung, welche bei der bedürftigen Person, welche die erfindungsgemäße Zusammensetzung einnimmt, eine Wirkung hervorruft. Die Dosierung erfolgt bevorzugt in den oben angegebenen Mengen. Die oben angegebenen Mengen sind bevorzugt für einen männlichen Homo Sapiens mit einem Körpergewicht von 75 kg ausgelegt. Männer mit höherem oder niedrigerem Körpergewicht sowie Frauen können entsprechend vom Dosisspektrum angepasst werden, wobei dies nicht notwendig ist. "Bedürftige Person" ist eine Person nach der Einnahme von Alkohol.

Die Erfindung soll nun anhand eines Ausführungsbeispiels näher erläutert werden, wobei dieses jedoch nur der Veranschaulichung dient und keinesfalls einschränkend auf den Umfang der Erfindung zu verstehen ist.

### Ausführungsbeispiele

### Beispiel 1:

| | | |
|---|---|---|
| NAC | 900 | mg |
| Magnesium | 600 | mg |
| Zink | 70 | mg |
| C | 1000 | mg |
| B1 | 100 | mg |
| B2 | 30 | mg |
| B3 als Niacinamid | 800 | mg |
| B5 | 60 | mg |
| B6 | 70 | mg |
| B12 | 500 | µg |
| Biotin | 600 | µg |
| Folsäure | 800 | µg |
| K1 | 160 | µg |
| A | 1000 | µg |
| E | 200 | mg |
| D3 | 10 | µg |
| Calcium | 300 | mg |
| Kupfer | 2 | mg |
| Selen | 75 | µg |
| Mangan | 2, 4 | mg |
| Kalium | 20 | mg |
| Eisen | 10 | mg |
| Jod | 220 | µg |
| Molybdän | 50 | µg |
| Chrom | 50 | µg |
| B3 als Niacin | 30 | mg |
| Taurin | - | mg |
| Methionin | - | mg |
| Tyrosin | - | mg |
| Inositol | - | mg |
| Cholin | - | mg |
| Coen. Q10 | 6 | mg |
| Glycin | - | mg |
| Glutamin | - | mg |
| Glutaminsäure | - | mg |
| Alpha-Liponsäure | - | mg |
| Coffein | - | mg |
| Carnitin | - | mg |

### Beispiel 2

| | | |
|---|---|---|
| NAC | 650 | mg |
| Magnesium | 300 | mg |
| Zink | 50 | mg |
| C | 1000 | mg |
| B1 | 100 | mg |
| B2 | 30 | mg |
| B3 als Niacinamid | 500 | mg |
| B5 | 60 | mg |
| B6 | 70 | mg |
| B12 | 1000 | µg |
| Biotin | 600 | µg |
| Folsäure | 600 | µg |
| K1 | 160 | µg |
| A | 400 | µg |
| E | 200 | mg |
| D3 | - | µg |
| Calcium | 300 | mg |
| Kupfer | 2 | mg |
| Selen | 50 | µg |
| Mangan | 2, 4 | mg |
| Kalium | - | mg |
| Eisen | 5 | mg |
| Jod | - | µg |
| Molybdän | 50 | µg |
| Chrom | - | µg |
| B3 als Niacin | 15 | mg |
| Taurin (separat morgens) | 800 | mg |
| Methionin | - | mg |
| Tyrosin (separat morgens) | 500 | mg |
| Inositol | - | mg |
| Cholin | - | mg |
| Coen. Q10 | - | mg |
| Glycin | - | mg |
| Glutamin | - | mg |
| Glutaminsäure | - | mg |
| Alpha-Liponsäure | - | mg |
| Coffein (separat | 75 | mg |
| morgens) | | |
| Carnitin (separat morgens) | - | mg |

### Beispiel 3

| | | |
|---|---|---|
| NAC | 1200 | mg |
| Magnesium | 400 | mg |
| Zink | 80 | mg |
| C | 500 | mg |
| B1 | 100 | mg |
| B2 | 30 | mg |
| B3 als Niacinamid | 800 | mg |
| B5 | 60 | mg |
| B6 | 70 | mg |
| B12 | 500 | µg |
| Biotin | 400 | µg |
| Folsäure | 800 | µg |
| K1 | - | µg |
| A | 1000 | µg |
| E | 100 | mg |
| D3 | - | µg |
| Calcium | 500 | mg |
| Kupfer | 2 | mg |
| Selen | 75 | µg |
| Mangan | 2 | mg |
| Kalium | - | mg |
| Eisen | 20 | mg |
| Jod | - | µg |
| Molybdän | 50 | µg |
| Chrom | - | µg |
| Niacin | 19 | mg |
| Taurin | - | mg |
| Methionin | 500 | mg |
| Tyrosin | - | mg |
| Inositol | 250 | mg |
| Cholin | 250 | mg |
| Coen. Q10 | - | mg |
| Glycin | | mg |
| Glutamin | 1000 | mg |
| Glutaminsäure | - | mg |
| Alpha-Liponsäure | | mg |
| Coffein | - | mg |
| Carnitin (separat morgens) | 500 | mg |

### Beispiel 4:

| | | |
|---|---|---|
| NAC | 700 | mg |
| Magnesium | 700 | mg |
| Zink | 100 | mg |
| C | 1000 | mg |
| B1 | 100 | mg |
| B2 | 30 | mg |
| B3 als Niacinamid | 600 | mg |
| B5 | 80 | mg |
| B6 | 70 | mg |
| B12 | 500 | µg |
| Biotin | 600 | µg |
| Folsäure | 800 | µg |
| K1 | - | µg |
| A | - | µg |
| E | 280 | mg |
| D3 | - | µg |
| Calcium | 300 | mg |
| Kupfer | 2 | mg |
| Selen | 100 | µg |
| Mangan | 2, 4 | mg |
| Kalium | 20 | mg |
| Eisen | 35 | mg |
| Jod | 100 | µg |
| Molybdän | 50 | µg |
| Chrom | 25 | µg |
| B3 als Niacin | - | mg |
| Taurin | 1000 | mg |
| Methionin | - | mg |
| Tyrosin | - | mg |
| Inositol | - | mg |
| Cholin | 200 | mg |
| Coen. Q10 | 3 | mg |
| Glycin | 500 | mg |
| Glutamin | - | mg |
| Glutaminsäure | 500 | mg |
| Alpha-Liponsäure | 50 | mg |
| Coffein | - | mg |
| Carnitin | - | mg |

Bei der Einnahme der Zusammensetzungen gemäß den Beispielen 1 bis 4 hat sich folgendes gezeigt:
- Wird die Zusammensetzung mit genügend Wasser vor der Alkoholeinnahme eingenommen, so wird die Person langsamer bzw. weniger betrunken bei gleicher Einnahme von Alkohol, als wenn die Person das Mittel nicht einnimmt. Ferner wird die Person, wenn sie das Mittel vor der Alkoholeinnahme eingenommen hat, während und nach der Alkoholeinnahme wieder schneller nüchtern. Dies ist unter anderem zurückzuführen auf die Verbesserung des Alkohol- und Acetaldehydstoffwechsels. Ferner wird die Person, wenn sie das Mittel vor der Alkoholeinnahme eingenommen hat, bei gleicher Einnahme von Alkohol, als wenn die Person das Mittel nicht einnimmt, geringere Symptome des so genannten Katers aufweisen. Sofern die Personen, die das Mittel vor der Alkoholeinnahme eingenommen hat, bei gleicher Menge Alkohol ohne Einnahme des Mittels einen Kater bekommen hätte, hat sich gezeigt, dass die Person bei gleicher Menge Alkohol nur einen geringen oder bis gar keinen Kater bekommen hat. Die körperliche und geistige Leistungsfähigkeit war am Tag nach der Alkoholeinnahme wesentlich weniger bis vernachlässigbar eingeschränkt.
- Wird die Zusammensetzung mit genügend Wasser nach der Alkoholeinnahme eingenommen, so wird die Person nach der Alkoholeinnahme schneller wieder nüchtern, als wenn die Person nach gleicher Alkoholeinnahme das Mittel nicht eingenommen hätte. Dies ist unter anderem zurückzuführen auf die Verbesserung des Alkohol- und Acetaldehydstoffwechsels. Ferner wird die Person, wenn sie das Mittel nach der Alkoholeinnahme eingenommen hat, bei gleicher Einnahme von Alkohol, als wenn die Person das Mittel nicht einnimmt, geringere Symptome des so genannten Katers aufweisen. Sofern die Personen, die das Mittel nach der Alkoholeinnahme eingenommen hat, bei gleicher Menge Alkohol ohne Einnahme des Mittels einen Kater bekommen hätte, hat sich gezeigt, dass die Person bei gleicher Menge Alkohol nach Einnahme des Mittels nur einen geringen oder bis gar keinen Kater bekommen hat. Die körperliche und geistige Leistungsfähigkeit war am Tag nach der Alkoholeinnahme wesentlich weniger bis vernachlässigbar eingeschränkt.
- Wird die Zusammensetzung mit genügend Wasser bei Vorhandensein eines Katers eingenommen, so hat sich gezeigt, dass die Symptome des Katers schneller verschwinden als wenn die Person das Mittel nicht eingenommen hätte. Sollte die Person noch spürbar intoxikiert sein, so wird sie nach Einnahme des Mittels schneller nüchtern (durch Abbau des "Restalkohols") als wenn die Person das Mittel nicht einnimmt. Die körperliche und geistige Leistungsfähigkeit verbessert sich nach Einnahme des Mittels schneller und besser als wenn die Person das Mittel nicht einnimmt.
- Ferner hat sich bei einer Testperson gezeigt, dass der Gewöhnungseffekt von Alkohol verringert oder verhindert werden kann. Das Mittel ist zwar nicht für den täglichen Dauergebrauch gedacht, aber diese Wirkung ist durchaus interessant und durchaus nachvollziehbar. Nach einer längeren Testreihe war der Effekt des Alkohols auf die Testperson gleichbleibend, insbesondere bei Einnahme des Mittels. Ferner traten die üblichen depressiven Verstimmungen, die nach längerem Alkoholgebrauch üblicherweise eintreten, nicht auf.
- Der Körper wird grundsätzlich durch die Einnahme des Mittels weniger durch Alkoholmissbrauch belastet, da die Leber wesentlich durch das Mittel unterstützt wird und eben weil relevante Mikronährstoffe in ausreichender Dosierung dem Körper zugeführt werden, so dass Mikronährstoffverlustfolgekrankheiten nicht eintreten.

## Patentansprüche

1. Zusammensetzung zur Reduzierung und/oder Vorbeugung von schädlichen Wirkungen von Alkoholkonsum umfassend in einer Dosisform 600-4000 mg einer Cysteinverbindung, ein pharmazeutisch annehmbares Magnesiumsalz, ein pharmazeutisch annehmbares Zinksalz, Vitamin B1, Vitamin B2, Vitamin B3, Vitamin B5, Vitamin B6 und Vitamin B12, wobei die Cysteinverbindung N-Acetylcystein ist.

2. Zusammensetzung gemäß Anspruch 1, **dadurch gekennzeichnet, dass** die Zusammensetzung zusätzlich ein pharmazeutisch annehmbares Calciumsalz und/oder Kupfer und/oder Selen und/oder Mangan und/oder Kalium und/oder Eisen und/oder Jod und/oder Molybdän und/oder Chrom enthält.

3. Zusammensetzung gemäß Anspruch 1 oder 2, **dadurch gekennzeichnet, dass** die Zusammensetzung zusätzlich Vitamin C und/oder Vitamin D3 und/oder Vitamin E und/oder Vitamin K1 und/oder Vitamin A und/oder Biotin enthält.

4. Zusammensetzung gemäß einem der Ansprüche 1 bis 3, **dadurch gekennzeichnet, dass** die Zusammensetzung zusätzlich Methionin und/oder Taurin und/oder Tyrosin und/oder Folsäure und/oder Cholin und/oder Coenzym Q10 und/oder Inositol und/oder Glycin und/oder Glutamin und/oder Glutaminsäure und/oder alpha-Liponsäure enthält.

5. Zusammensetzung gemäß einem der Ansprüche 1 bis 4, **dadurch gekennzeichnet, dass** die Zusammensetzung zusätzlich Wasser enthält.

6. Zusammensetzung gemäß einem der Ansprüche 1 bis 5, **dadurch gekennzeichnet, dass** es sich um eine oral oder intravenös verabreichbare Zusammensetzung handelt.

7. Zusammensetzung gemäß einem der Ansprüche 1 bis 6 zur Verwendung in einem Verfahren zur Reduzierung und/oder Vorbeugung von schädlichen Wirkungen von Alkoholmissbrauch, wobei das Verfahren die Verabreichung einer wirksamen Menge der Zusammensetzung an eine bedürftige Person umfasst.

## Claims

1. A composition for reducing and/or preventing harmful effects of alcohol consumption comprising in a dosage form 600-4000 mg of a cysteine compound, a pharmaceutically acceptable magnesium salt, a pharmaceutically acceptable zinc salt, vitamin B1, vitamin B2, vitamin B3, vitamin B5, vitamin B6 and vitamin B12, wherein the cysteine compound is N-acetylcysteine.

2. Composition according to claim 1, **characterized in that** the composition additionally contains a pharmaceutically acceptable calcium salt and/or copper and/or selenium and/or manganese and/or potassium and/or iron and/or iodine and/or molybdenum and/or chromium.

3. Composition according to claim 1 or 2, **characterized in that** the composition additionally contains vitamin C and/or vitamin D3 and/or vitamin E and/or vitamin K1 and/or vitamin A and/or biotin.

4. Composition according to any one of claims 1 to 3, **characterized in that** the composition additionally contains methionine and/or taurine and/or tyrosine and/or folic acid and/or choline and/or coenzyme Q10 and/or inositol and/or glycine and/or glutamine and/or glutamic acid and/or alpha-lipoic acid.

5. Composition according to any one of claims 1 to 4, **characterized in that** the composition additionally contains water.

6. Composition according to any one of claims 1 to 5, **characterized in that** it is an orally or intravenously administrable composition.

7. A composition according to any one of claims 1 to 6 for use in a method of reducing and/or preventing the harmful effects of alcohol abuse/misuse, the method comprising administering an effective amount of the composition to a person in need.

## Revendications

1. Composition pour réduire et/ou prévenir les effets nocifs de la consommation d'alcool comprenant, sous forme de dose, 600-4000 mg d'un composé de cystéine, un sel de magnésium pharmaceutiquement acceptable, un sel de zinc pharmaceutiquement acceptable, de la vitamine B1, de la vitamine B2, de la vitamine B3, de la vitamine B5, de la vitamine B6 et de la vitamine B12, dans laquelle le composé de cystéine est la N-acétylcystéine.

2. Composition selon la revendication 1, **caractérisée en ce que** la composition contient en outre un sel de calcium et/ou du cuivre et/ou du sélénium et/ou du manganèse et/ou du potassium et/ou du fer et/ou de l'iode et/ou du molybdène et/ou du chrome pharmaceutiquement acceptables.

3. Composition selon la revendication 1 ou 2, **caractérisée en ce que** la composition contient en outre de la vitamine C et/ou de la vitamine D3 et/ou de la vitamine E et/ou de la vitamine K1 et/ou de la vitamine A et/ou de la biotine.

4. Composition selon l'une des revendications 1 à 3, **caractérisée en ce que** la composition contient en outre de la méthionine et/ou de la taurine et/ou de la tyrosine et/ou de l'acide folique et/ou de la choline et/ou du coenzyme Q10 et/ou de l'inositol et/ou de la glycine et/ou de la glutamine et/ou de l'acide glutamique et/ou de l'acide alpha-lipoïque.

5. Composition selon l'une des revendications 1 à 4, **caractérisée en ce que** la composition contient en outre de l'eau.

6. Composition selon l'une des revendications 1 à 5, **caractérisée en ce qu'**il s'agit d'une composition administrable par voie orale ou intraveineuse.

7. Composition selon l'une des revendications 1 à 6, destinée à être utilisée dans une méthode de réduction et/ou de prévention des effets nocifs de l'abus d'alcool, ladite méthode comprenant l'administration d'une quantité efficace de ladite composition à une personne dans le besoin.
